Europäisches Patentamt

European Patent Office

Office européen des brevets

(19)

(11) Publication number: **0 322 852**
**A1**

# (12) EUROPEAN PATENT APPLICATION

(21) Application number: **88121697.2**

(22) Date of filing: **27.12.88**

(51) Int. Cl.⁴: **A61B 5/04 , A61N 1/04**

(30) Priority: **29.12.87 JP 201054/87**

(43) Date of publication of application:
**05.07.89 Bulletin 89/27**

(84) Designated Contracting States:
**DE FR GB IT**

(71) Applicant: **FUKUDA DENSHI CO., LTD.**
**39-4, Hongo 3-chome Bunkyo-ku**
**Tokyo 113(JP)**

Applicant: **RIKEN CO. LTD.**
**1-13-5 Kudankita**
**Chiyoda-ku Tokyo(JP)**

(72) Inventor: **Shimizu, Chuji c/o Fukuda Denshi**
**Co. Ltd.**
**Hongo Enterprise Place 2-35-8 Hongo**
**Bunkyo-ku Tokyo(JP)**
Inventor: **Onodera, Yasuaki**
**c/o Fukuda Denshi Co. Ltd. 3-39-4 Hongo**
**Bunkyo-ku Tokyo(JP)**
Inventor: **Minagawa, Sakae c/o Riken Co. Ltd.**
**Kumagaya Enterprise Place 810 Kumagaya**
**Kumagaya-shi Saitama(JP)**
Inventor: **Kawamoto, Hiroshi c/o Riken Co.**
**Ltd.**
**Kumagaya Enterprise Place 810 Kumagaya**
**Kumagaya-shi Saitama(JP)**
Inventor: **Koike, Masao c/o Riken Co. Ltd.**
**Kumagaya Enterprise Place 810 Kumagaya**
**Kumagaya-shi Saitama(JP)**
Inventor: **Ishii, Hiroyoshi c/o Riken Co. Ltd.**
**Kumagaya Enterprise Place 810 Kumagaya**
**Kumagaya-shi Saitama(JP)**

(74) Representative: **Behn, Klaus, Dipl.-Ing.**
**Patentanwalt Lindenberg 34**
**D-8134 Pöcking bei München(DE)**

(54) Lead electrode for using with the living body.

(57) A lead electrode for use with the living body comprises an electrode element (1) provided with a plastic substrate member (14), composed of a head portion (11) having partially a dent portion (11a) in flatness, a neck portion (12) thinner than said head portion and having a straight portion communicated with said dent portion, and a bottom portion (13) thicker than said neck portion. The outer surface of the plastic substrate member is coated by a layer of metallic powder (15). A rigid supporting member (3) with which said head and neck portions are engaged, has an opening in the center of it and an adhesive member (2, 2a) mounted on the surrounding of said opening. Adhesive agent (2a) is applied to the back surface of the adhesive member (2).

# Fig. 3

## LEAD ELECTRODE FOR USING WITH THE LIVING BODY

### BACKGROUND OF THE INVENTION

#### 1. Field of the Invention

The present invention relates to lead electrode for using with the living body. More particularly, it relates to lead electrode used when weak voltage is led out of the living body.

#### 2. Description of the Related Art

It is well known that the living body is constituted by the heart, brain etc., and the activity thereof induces voltage.

In the above elements constituting the living body, the heart generates weak voltage which is recorded in the electrocardiograph setted outside of the living body, in order to serve as data for diagnosing whether or not the heart is abnormal.

In this case, it is necessary to contact closely a lead electrode with the skin surface of the living body so as to connect electrically the input portion of the electrocardiograph with the living body.

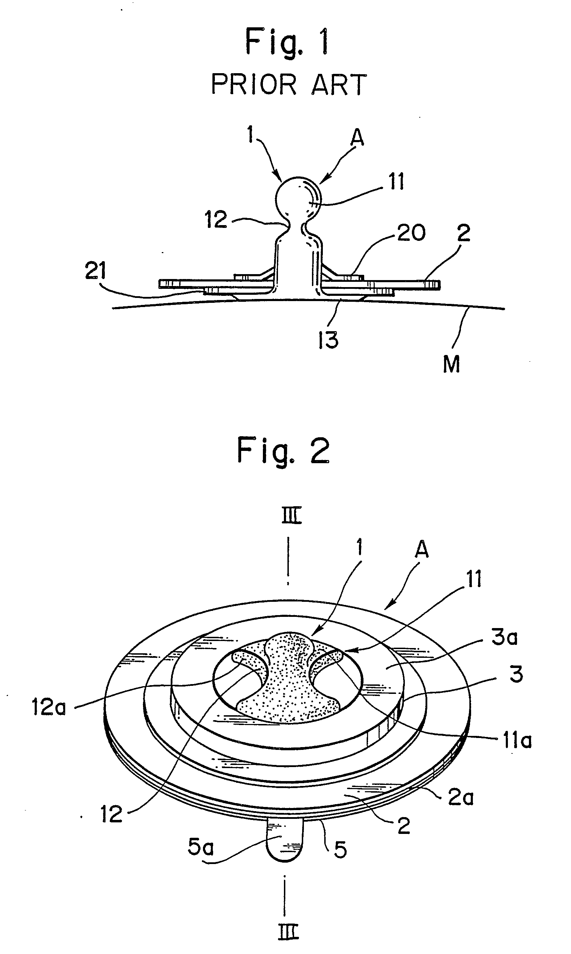

Figure 1 is an explanatory drawing of the conventional lead electrode for using with the living body.

In Fig. 1, a lead electrode A has an electrode element 1 made of metal, with which electrode element 1 a substrate member 2 is engaged.

The substrate member 2 combines expansion and contraction, which member 2 is pinched by members 20 and 21, and thereby is fixed to the electrode element 1.

The electrode element 1 is provided with a bottom portion 13 which is formed broadly so as to contact closely with the skin surface M of the living body, whereby weak voltage is led out.

The electrode element 1 is the most important of the elements constituting the lead electrode A, because it leads the above weak voltage from the skin surface M of the living body to the electrocardiograph through a terminal (omitted in Fig. 1) of the lead line mounted on a head portion 11 of the electrode element 1.

However, the problems of the conventional lead electrode are as follows..

(1) When the terminal of the lead line is shifted by some external pressure, the electrode element is also accordingly shifted.

The result is that the lead electrode A falls apart the skin surface M of the living body, and thereby it is difficult to measure exactly the electrocordiogram.

(2) Since the electrode element 1 is produced by plating with silver, the manufacturing process thereof is complex and consequently the cost is expensive.

### SUMMARY OF THE INVENTION

An object of the present invention is to fix the electrode element firmly even if the terminal of the lead line is shifted, whereby it is possible to measure exactly the electrocordiogram.

Another object of the present invention is to produce the electrode element by means of the simpler manufacturing process, whereby the cost is lower.

The above-mentioned objects can be achieved by lead electrode for using with the living body comprising an electrode element provided with a plastic substrate member composed of a head portion having partially a dent portion in flatness, a neck portion thinner than said head portion and having a straight portion communicated with said dent portion, and a bottom portion thicker than said neck portion, on the outer surface of which plastic substrate member metallic powder is coated, a rigid supporting member with which said head and neck portions and are engaged, in the center of which rigid supporting member an opening is formed, and an adhesive member mounted on the surrounding of said opening, to the back surface of which adhesive member adhesive agent is applied.

### BRIEF DESCRIPTION OF THE DRAWINGS

Further features and advantages of the present invention will be apparent from the ensuring description with reference to the accompanying drawings, wherein:

Fig. 1 is an explanatory drawing of the conventional lead electrode for using with the living body;

Fig. 2 is a perspective drawing of an embodiment of the present invention;

Fig. 3 is a sectional drawing taken on line III - III of Fig. 2;

Fig. 4 is a perspective drawing of another embodiment of the present invention;

Fig. 5 is a sectional drawing taken on line V - V of Fig. 4;

Fig. 6a is an explanatory drawing wherein the terminal of the lead line is mounted on the lead electrode for using with the living body;

Fig. 6b is a perspective drawing of the terminal of the lead line.

DESCRIPTION OF THE PREFERRED EMBODIMENTS

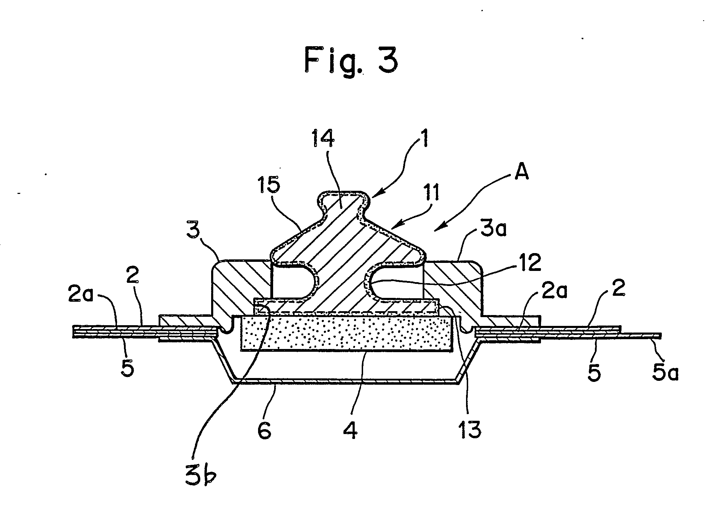

Fig. 2 is a perspective drawing of an embodiment of the present invention, and Fig. 3 is a sectional drawing taken on line III - III of Fig. 2.

Reference numeral A shows a lead electrode for using with the living body, which lead electrode has an electrode element 1, an adhesive member 2, and a rigid supporting member 3.

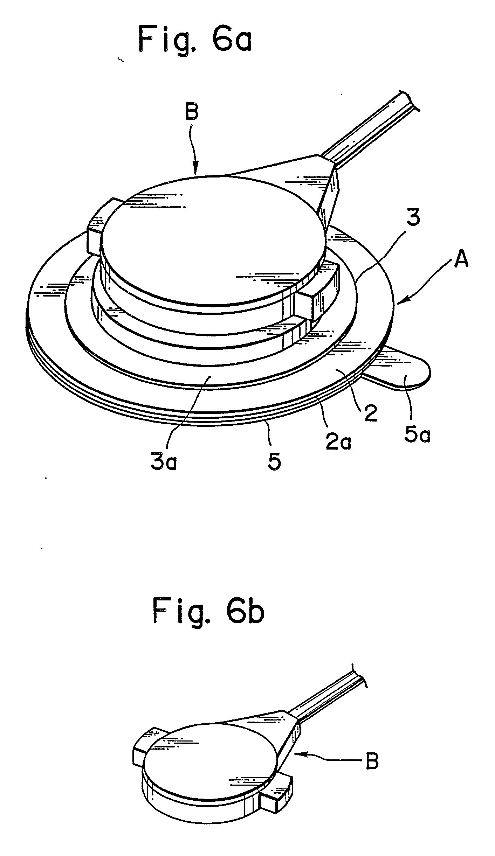

The electrode element 1 is provided with a plastic substrate member 14 composed of a head portion 11, a neck portion 12, and a bottom portion 13 so as to connect more closely with the terminal B (see Fig.6a) of the lead line.

The plastic substrate member 14 has a coating layer 15 of metallic powder of silver, amorphous alloy etc., in order to be provided with the electric conduction, which member 14 is made of plastics because it is produced by means of the simpler manufacturing process, and accordingly it is suitable for mass production, whereby the cost becomes expensive. And, it is because that since plastics is generally light, the lead electrode A is capable of mounted stably on the skin surface of the living body. Moreover, it is because that the thermal conduction is reduced, and that coldness is not felt when the lead electrode A is mounted on the skin surface of the living body.

The head portion 11 has partially a dent portion 11a in flatness, and the neck portion 12 is thinner the head portion 11 and has a straight portion 12a communicated with the dent portion 11a.

The dent portion 11a and straight portion 12a are disposed because metallic powder is small in quantity, in case of coating.

The bottom portion 13 is thicker than the neck portion 12, to the back surface of which bottom portion 13 a supplementary member 4 is bonded.

The supplementary member 4 is formed by gelhydrate layer with adhesion and conductivity of gelatine, agar, and polyacrylic amid etc., alternately it is formed by polyurethan form layer, through which supplementary member 4 weak voltage is lead to the electrode element 1, when it is contacted closely with the skin surface of the living body.

When the bottom portion 13 is directly contacted closely with the skin surface of the living body, weak voltage is not capable of measured exactly owing to the contact resistance between the bottom portion 13 and the skin surface. Hence, after cream is applied to the skin surface so as to reduce the contact resistance , the bottom portion 13 is contacted closely with the applied cream, whereby weak voltage should be measured.

However, it is troublesome to apply cream to the skin surface of the living body whenever weak voltage is measured.

Thereby, the supplementary member 4 is bonded to the back surface of the bottom portion 13 instead of the above cream so as to remove the trouble.

The rigid supporting member 3 is formed by moulding high polymer with injection moulding.

An opening 3b is formed in the center of the rigid supporting member 3, through which opening 3b the bottom portion 13 runs.

The head portion 11 and the bottom portion 13 are engaged with the rigid supporting member 3, and thereby even if the terminal B (see Figs.6a and 6b) of the lead line is shifted by some external pressure, the electrode element 1 is fixed firmly.

The result is that the lead electrode A does not fall apart the skin surface of the living body, and the electrocordiogram is accordingly capable of measured exactly.

The adhesive member 2 is mounted on the surrounding of the opening 3b, to the back surface of which adhesive member 2 adhesive agent 2a is applied.

The adhesive member 2 is annular, combines expansion and contraction, and is formed by electrolyte with ionic conduction, which adhesive member 2 is made of, for example, high polymerized nonwoven fabric.

A separator 5 is bonded to the back surface of the adhesive member 2 with adhesion agent 2a, on the back surface of which separator 5 a cover 6 is mounted so as to protect the lead electrode A.

When the lead electrode A is used actually, the separator 5 is taken off from the adhesive member 2 by pinching a grip 5a with fingers, and thereby the cover 6 is also removed.

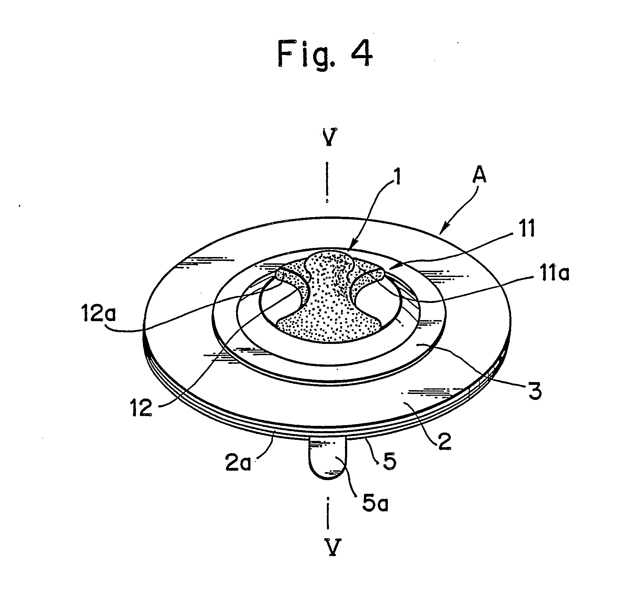

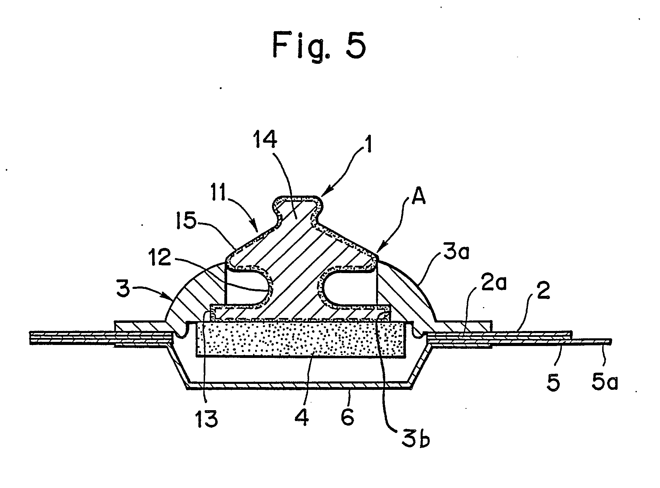

Fig. 4 is a perspective drawing of another embodiment of the present invention, and Fig. 5 is a sectional drawing taken on line V - V of Fig. 4.

The embodiment of Fig. 4 only differs from that of Fig. 2 in the form of an upper surface 3a of the rigid supporting member 3.

That is to say, the upper surface 3a is square as shown in Fig. 3, but the upper surface 3a is round as shown in Fig. 5.

According to the present invention, the head portion 11 and the bottom portion 13 are engaged with the rigid supporting member 3, and thereby even if the terminal B (see Figs.6a and 6b) of the lead line is shifted by some external pressure, the electrode element 1 is fixed firmly.

Hence, the present invention has the effect that the lead electrode A does not fall apart the skin surface of the living body, and that the electrocordiogram is accordingly capable of measured exactly.

Moreover, according to the present invention, the plastic substrate member 14 has a coating layer 5 of metallic powder of silver, amorphous alloy etc., in order to be provided with the electric conduction, which member 14 is made of plastics.

Hence, the present invention has the effect that since the plastic substrate member 14 is produced by means of the simpler manufacturing process compare with the conventional manufacturing process for plating with silver, and that accordingly it is suitable for mass production, whereby the cost becomes expensive.

And, owing to the plastic substrate member 14, the additional effects of the present invention are as follows.

That is to say, since plastics is generally light, the lead electrode A is capable of mounted stably on the skin surface of the living body. Moreover, the thermal conduction is reduced, and coldness is not felt when the lead electrode A is mounted on the skin surface of the living body.

**Claims**

1. Lead electrode for using with the living body comprising:

an electrode element (1) provided with a plastic substrate member (14) composed of a head portion (11) having partially a dent portion (11a) in flatness, a neck portion (12) thinner than said head portion (11) and having a straight portion (12a) communicated with said dent portion (11a), and a bottom portion (13) thicker than said neck portion (12), on the outer surface of which plastic substrate member (14) metallic powder is coated,

a rigid supporting member (3) with which said head and neck portions (11) and (12) are engaged, in the center of which rigid supporting member (3) an opening (3b) is formed, and

an adhesive member (2) mounted on the surrounding of said opening (3b), to the back surface of which adhesive member (2) adhesive agent (2a) is applied.

2. Lead electrode for using with the living body according to claim 1, wherein said adhesive member (2) is annular, combines expansion and contraction, and is formed by electrolyte with ionic conduction.

3. Lead electrode for using with the living body according to claim 1, wherein said adhesive member (2) is made of high polymerized nonwoven fabric.

4. Lead electrode for using with the living body according to claim 1, wherein said rigid supporting member (3) is formed by moulding high polymer with injection moulding.

5. Lead electrode for using with the living body according to claim 1, wherein a supplementary member 4 is bonded to the back surface of saide bottom portion 13.

Fig. 1
PRIOR ART

Fig. 2

EP 0 322 852 A1

# Fig. 3

# Fig. 4

# Fig. 5

# Fig. 6a

# Fig. 6b

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | US-A-4 029 086 (E.R. CORASANTI) <br> * Abstract; column 2, line 49 - column 3, line 65; figures 1-4 * | 1,2,5 | A 61 B 5/04 <br> A 61 N 1/04 |
| Y | US-A-4 270 544 (S.J. GILDEN et al.) <br> * Abstract; column 2, line 29 - column 3, line 18; figures 1,2 * | 1,2,5 | |
| A | US-A-3 993 049 (J.A.R. KATER) <br> * Abstract; column 3, line 46 - column 4, line 63; figures 1-6 * | 1-3 | |
| A | US-A-3 882 853 (J.W. GOFMAN et al.) <br> * Abstract; column 4, lines 33-35; column 4, line 48 - column 5, line 22; figures 1-3 * | 1-5 | |

**TECHNICAL FIELDS SEARCHED (Int. Cl.4)**

A 61 B
A 61 N

The present search report has been drawn up for all claims

| Place of search | Date of completion of the search | Examiner |
|---|---|---|
| THE HAGUE | 30-03-1989 | HUNT,B.W. |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

................................................................

& : member of the same patent family, corresponding
document

EPO FORM 1503 03.82 (P0401)